# FASCICULE DE BREVET EUROPEEN

(11) **EP 3 794 381 B1**
(45) Date de publication et mention de la délivrance du brevet: **06.07.2022**
(21) Numéro de dépôt: 19728891.3
(22) Date de dépôt: 17.05.2019
(51) Int. Cl.: G01T 1/29

(54) **SYSTÈME ET PROCÉDÉ D'IMAGERIE PAR DÉTECTION DE RAYONNEMENTS GAMMA**
BILDGEBUNGSSYSTEM UND VERFAHREN AUF DER BASIS VON GAMMASTRAHLUNGSDETEKTION
IMAGING SYSTEM AND METHOD BASED ON GAMMA RADIATION DETECTION

(30) Priorité: 18.05.2018 FR 1854192
(43) Date de publication de la demande: 24.03.2021
(73) Titulaire: Agence Nationale pour la Gestion des Déchets Radioactifs, 92298 Châtenay-Malabry (FR); Damavan Imaging, 10430 Rosières Près Troyes (FR)
(72) Inventeur: ILTIS, Alain, 10000 TROYES (FR)
(74) Mandataire: Debay, Damien
(86) Numéro de dépôt international: PCT/EP2019/062805
(87) Numéro de publication internationale: WO 2019/219912

(56) Documents cités:
- WO-A1-2016/185123
- WO-A1-2017/025842
- WO-A1-2018/019941
- A. ILTIS ET AL: "Temporal Imaging CeBr 3 Compton Camera: A New Concept for Nuclear Decommissioning and Nuclear Waste Management", EPJ WEB OF CONFERENCES, vol. 170, 10 janvier 2018 (2018-01-10), page 06003, XP055544293, ISSN: 2101-6275, DOI: 10.1051/epjconf/201817006003
- A BRAEM ET AL: "Feasibility of a novel design of high resolution parallax-free Compton enhanced PET scanner dedicated to brain research", PHYSICS IN MEDICINE AND BIOLOGY, vol. 49, no. 12, 21 juin 2004 (2004-06-21) , pages 2547-2562, XP055544314, Bristol GB ISSN: 0031-9155, DOI: 10.1088/0031-9155/49/12/006

## Description

### DOMAINE TECHNIQUE DE L'INVENTION

La présente demande se rapporte au domaine de l'imagerie et plus particulièrement à l'imagerie de sources de rayons gamma. En particulier, l'invention concerne un système et des procédés d'imagerie par détection de rayons gamma combinant la détection de type caméra Compton et la détection de coïncidence de type Tomographie à Emission de Positron (PET). L'invention concerne en outre l'utilisation du système d'imagerie et/ou de détection dans les domaines notamment de l'astronomie, de l'industrie nucléaire et du médical.

### ARRIERE-PLAN TECHNOLOGIQUE DE L'INVENTION

A l'heure actuelle, l'imagerie de sources de rayons gamma (dont l'énergie est généralement supérieure à 30 KeV) s'effectue essentiellement à des fins de diagnostic médical autour de trois techniques : Le PET, le SPECT et la Caméra Compton.

Le SPECT est basée sur la scintigraphie et permet de réaliser des images et des reconstructions en trois dimensions des organes et de leur métabolisme au moyen d'un ensemble de gamma caméras tournant autour d'un patient. Le SPECT peut utiliser plusieurs énergies de rayons gamma, mais le collimateur en plomb qui permet de connaître la trajectoire des rayons en absorbe plus de 99%.

Le PET utilise généralement un anneau de détecteurs segmentés. Pour le PET on utilise des composés radiopharmaceutiques émetteurs de positrons. Ceux-ci donnent naissance à une paire de photons de 511 KeV dont il est possible de localiser la source d'émission grâce à leur détection simultanée sur l'anneau de détecteurs (détection de coïncidence). Cependant les radioéléments utilisés pour le PET ont une vie brève et sont donc souvent coûteux. L'imagerie PET est une imagerie fonctionnelle qui est très attractive pour guider une intervention médicale par une image qui indique clairement où se situe la source du rayonnement que l'on observe. C'est le cas en particulier en Oncologie où l'émission des rayons est concentrée sur les tumeurs et permet de différencier ces dernières des tissus sains. De plus, l'imagerie PET est également largement utilisée en imagerie préclinique sur le rat ou sur la souris pour observer in vivo les processus biologiques. L'imagerie PET est d'ailleurs la technologie d'imagerie qui permet d'obtenir les images les plus précises actuellement (rapport signal/bruit et résolution angulaire), pour des rayonnements gamma énergétiques de 511 KeV. En particulier, un des points clefs dans l'imagerie PET est la mesure précise du temps de vol des photons 511 KeV depuis leur lieu d'émission. Cette mesure de temps de vol est d'autant meilleure que l'épaisseur de scintillateur traversée est mince. Cependant, dans de telles configurations, une grosse part des photons n'est pas détectée correctement car la probabilité de détection croît avec l'épaisseur de scintillateur traversé. Cependant, les imageurs PET sont habituellement constitués d'un anneau complet autour du patient (ou tout objet) dont le diamètre est d'environ 80 cm pour une largeur de plus de 20 cm et une épaisseur de scintillateur de 20 mm. Cette configuration en anneau est imposée par le champ de vue très réduit du PET. En effet, seules les interactions en coïncidence entre deux détecteurs sont observées, ce qui impose un angle solide réduit. Cette configuration rend leur utilisation difficilement envisageable dans un contexte d'opération chirurgicale. Par ailleurs, les scanners PET sont très onéreux (de l'ordre de deux millions d'euros) du fait du volume de détecteurs nécessaires. D'autre part, dans le contexte de l'imagerie préclinique ou les dimensions du scanner sont beaucoup plus réduites, les doses de radioactivité injectées aux souris sont très importantes ce qui peut perturber les processus physiologiques que l'on souhaite observer. De même, pour une imagerie interventionnelle, il serait intéressant de pouvoir réduire de manière drastique la dose injectée (par exemple une dose inférieure ou égale à 1MBq) au voisinage de l'organe à traiter.

La caméra Compton, comme le SPECT, permet de faire une image quelle que soit l'énergie du rayonnement gamma, mais contrairement au SPECT, tous les photons peuvent contribuer à l'image. Cependant, les applications de la caméra Compton sont aujourd'hui encore souvent limitées, notamment à cause de son coût, du niveau élevé de bruit et de la difficulté d'obtenir des reconstructions précises. Plus généralement dans toutes les technologies ci-dessus, lorsqu'on utilise des cristaux scintillants pour réaliser une image de sources de rayonnement gamma, on se heurte à la nature probabiliste de l'interaction photon gamma/matière. Nous remarquons essentiellement deux effets. Le premier effet réside dans le fait que le photon gamma peut être absorbé à une profondeur quelconque sur son trajet de propagation (Effet « Depth of Interaction » selon la terminologie anglo-saxonne). Le deuxième effet consiste en ce que tous les systèmes d'imagerie actuels (matrice de pixels ou caméra d'Anger) reposent sur le postulat que l'endroit où a lieu le maximum d'émission de lumière est le lieu où le photon gamma a été détecté. Du fait de la déviation Compton, ce postulat est correct tant que l'on considère la valeur moyenne d'un grand nombre d'événements. Par contre, dans le cas d'un scanner de type PET, si l'on reconstitue la position d'un événement unique, l'erreur, sur la position, peut être de plusieurs millimètres. La solution adoptée est alors de rejeter les événements pour lesquels l'énergie déposée n'est pas correcte. Cela conduit à rejeter un grand nombre d'événements. Des solutions, dites « d'imagerie temporelle » ont donc été développées, comme décrit notamment dans les demandes de brevets français FR2997766 et FR3013125 en ce qui concerne le PET. De plus, concernant la technologie des caméras Compton, les demandes de brevet WO2016185123 et WO2017077164 décrivent des systèmes et procédés qui tirent avantages à la fois de l'imagerie temporelle, mais également de la combinaison de l'imagerie Compton et de l'imagerie PET.

L'état de la technique comprend également la demande de brevet WO 2018/019941 A1 qui divulgue des exemples de systèmes et de méthodes de surveillance de l'activité métabolique et de détecteurs de photons.

La présente demande propose donc des solutions pour pallier à certains des inconvénients de l'art antérieur, notamment en visant à fournir, de préférence à moindre coût, des dispositifs, systèmes et procédés dont l'utilisation est la plus variée possible et/ou qui permettent d'améliorer la qualité des images obtenues.

Par ailleurs, une autre problématique générale et constante dans le domaine concerne la mesure du temps d'arrivée des premiers photons sur un pixel donné, car elle est importante pour la qualité des images obtenues à l'aide des signaux recueillis par les scanners PET et également pour les camera Compton temporelle. En particulier, le premier photon détecté dans un cristal permet de mesurer la coïncidence temporelle pour une émission à 511 KeV et permet donc d'estimer le temps de vol des photons depuis la source (« Time of Flight » selon la terminologie anglo-saxonne). De plus, dans une logique d'imagerie temporelle, le temps d'arrivée des premiers photons sur chaque pixel permet de déterminer la position du disque (et donc du cône) des photons non diffusés, ce qui permet d'améliorer la précision de l'estimation de la position (notamment en « profondeur », c'est-à-dire parallèlement à l'axe d'imagerie sur lequel sont alignés les détecteurs PET).

L'utilisation de cristaux monolithiques pour des scanners PET présente de multiples avantages, tels qu'un accès aisé à la mesure de la profondeur, un coût réduit et un potentiel de résolution spatiale élevée, notamment en utilisant les informations relatives à la distribution des événements dans le temps (« imagerie temporelle », comme mentionné ci-dessus). Les cristaux scintillants les plus rapides sont aujourd'hui les halogénures de Lanthane, comme par exemple le CeBr3 ou le LaBr3 : Ce qui émettent jusqu'à 4 fois plus de photons pendant la première nanoseconde que le LYSO : Ce souvent utilisé actuellement dans le domaine. À ce titre, ces halogénures de Lanthane sont les meilleurs candidats pour l'imagerie temporelle. Cependant ces cristaux sont très délicats à mettre en œuvre car ils sont extrêmement sensibles aux conditions atmosphériques (notamment l'hydrométrie), ce qui restreint leur utilisation sous la forme de cristaux monolithiques.

Néanmoins, l'utilisation des cristaux monolithiques se heurte à un paradoxe en ce qui concerne la mesure du temps d'arrivée des photons. En effet, a priori, la mesure du temps d'arrivée des premiers photons devrait être plus précise dans un cristal monolithique que dans un détecteur sous la forme d'une matrice (i.e., pixelisée) de cristaux scintillants, notamment du fait de l'absence de déviation optique depuis le lieu d'émission. Pourtant, dans un détecteur « pixelisé », le premier photon détecté par un pixel donné est très susceptible d'avoir subi de multiples réflexions sur les faces latérales du cristal avant d'être collecté par le photodétecteur et l'information sur le temps réel d'émission sera donc imprécise, voire totalement perdue. En revanche, dans un cristal monolithique, une fois la position de l'interaction connue, il est possible de déterminer un « disque des photons non diffusés » dans lequel la majorité des photons détectés n'a subi aucune perturbation (e.g., réflexion) entre leurs points d'émission dans la source et leurs points de collecte dans le photodétecteur. Le temps d'arrivée est par conséquent plus précis. Aujourd'hui, cependant, les résolutions temporelles mesurées avec des cristaux monolithiques sont inférieures à celles qui sont mesurées avec des réseaux de pixels.

La présente demande propose donc également des solutions à ce problème paradoxal.

### DESCRIPTION GENERALE DE L'INVENTION

Un but de la présente invention est de pallier au moins certains inconvénients de l'art antérieur en proposant un système d'imagerie de rayons gamma qui est peu encombrant et qui est utilisable dans de nombreuses conditions, notamment en imagerie préclinique ou en imagerie clinique d'un objet ou organe spécifique, ou même pour réaliser des biopsies guidées par imagerie.

Ce but est atteint par un système d'imagerie par détection de rayonnement gamma , selon la revendication indépendante 1, comprenant au moins une unité de traitement, au moins un châssis, et au moins un ensemble de modules de détection montés sur ledit au moins un châssis et fournissant au moins un signal analysé par ladite au moins une unité de traitement, chacun des modules comprenant au moins un scintillateur émettant des photons, lorsqu'il est soumis à l'influence dudit rayonnement gamma, au moins un photodétecteur générant ledit signal en réponse auxdits photons émis par ledit scintillateur et au moins un dispositif d'acquisition qui recueille ledit signal pour le transmettre à ladite unité de traitement, le système étant caractérisé en ce que:
- ledit châssis comprend une pluralité d'emplacements répartis selon au moins une portion de deux calottes sphériques en vis à vis l'une de l'autre et définissant un volume d'imagerie à l'intérieur du système, lesdites calottes étant séparées d'une distance permettant d'accueillir un objet à imager dans ledit volume ;
- ledit châssis étant ouvert, sur au moins un coté entre les deux calottes, de sorte à laisser l'accès libre audit objet à imager dans ledit volume ;
- ledit ensemble de modules de détection comporte au moins un module de type caméra Compton, possédant un champ de visée dirigé vers un volume délimité par ledit châssis, et dont le scintillateur comprend au moins une plaque de cristal scintillateur, dit rapide, dont le temps de montée au pic de lumière est inférieur à 1ns, de sorte que ce module est apte à produire une image en utilisant la diffusion Compton ;
- ledit ensemble de modules de détection comporte au moins un couple de modules PET de détection de coïncidences, diamétralement opposés l'un par rapport à l'autre sur ledit châssis, chacun sur l'une des deux calottes, et définissant un axe d'imagerie.
- ladite unité de traitement analyse le signal issu dudit module de type Compton pour déterminer l'intersection dudit axe d'imagerie avec ledit champ de visée et déterminer les orientations et/ou emplacements optimaux des divers modules de détection au sein desdites calottes du châssis pour que l'axe d'imagerie passe par la source dudit rayonnement gamma dans ledit objet à imager.

Selon une autre particularité, le système comporte au moins un moteur, piloté par ladite unité de traitement et contrôlant la mobilité d'au moins un desdits modules de détection ledit châssis et/ou de l'objet à observer à l'intérieur dudit volume pour obtenir lesdites orientations et/ou lesdits emplacements optimaux définis par ladite unité de traitement.

Selon une autre particularité, au moins un desdits ensembles de modules de détection comporte en fait seulement deux modules, grâce à un premier module, dit "hybride", dont le scintillateur comprend au moins une plaque de cristal scintillateur, dit rapide, dont le temps de montée au pic de lumière est inférieur à 1ns, ledit module "hybride" étant apte à produire à la fois une diffusion Compton et une absorption d'au moins une partie du rayonnement gamma pour une détection de coïncidence entre les événements dans ce premier module hybride et les événements dans un second module de détection avec lequel ce premier module hybride forme donc ledit couple de modules PET de détection de coïncidences.

Selon une autre particularité, ledit second module de détection de chaque ensemble est soit un module hybride également, soit un module, dit "PET", apte à produire seulement une absorption photoélectrique pour une détection de coïncidence d'événements par rapport à l'autre module de détection avec lequel il forme ledit couple de modules PET de détection de coïncidences.

Selon une autre particularité, chaque ensemble comporte plusieurs seconds modules en vis-à-vis du premier module hybride qui est orientable par ledit moteur pour aligner l'axe d'imagerie entre ledit module hybride et l'un ou l'autre de ces seconds modules.

Selon une autre particularité, ledit moteur est apte à déplacer au moins ledit second module sur ledit châssis, pour le placer en vis-à-vis du premier module hybride qui est orienté par ledit moteur pour aligner l'axe d'imagerie entre le premier et le second module.

Selon une autre particularité, le premier et le second module sont tous les deux des modules hybrides dont au moins un d'entre eux est orientable et au moins un d'entre eux est déplaçable sur le châssis, par ledit moteur.

Selon une autre particularité, ladite unité de traitement du signal analyse les signaux issus de chaque ensemble de modules de détection et détermine à la fois les événements de diffusion Compton et les événements d'absorption photoélectrique, pour obtenir la position tridimensionnelle, l'énergie et la séquence temporelle des interactions, Compton et photoélectriques, du rayonnement gamma dans le volume entre les modules de détection de chaque ensemble.

Selon une autre particularité, l'unité de traitement du signal mesure, dans le signal issu du module hybride, un seuil temporel de coïncidence dans une fenêtre temporelle inférieure au temps de transfert maximum de la lumière dans le module hybride, à l'intérieur de la plaque unique ou entre les plaques et, pour identifier des événements Compton valides.

Selon une autre particularité, l'unité de traitement calcule le temps de vol des photons entre les deux modules du couple de module PET premier module hybride et le second module pour mesurer la profondeur de la source de rayonnement gamma dans l'objet à imager.

Selon une autre particularité, l'unité de traitement utilise le signal d'au moins un couple module de type caméra Compton pour combiner leurs imageries Compton et réaliser une triangulation afin de mesurer la profondeur de la source de rayonnement gamma dans l'objet à imager.

Selon une autre particularité, le système est caractérisé en ce que :
- ledit scintillateur est un cristal scintillateur monolithique ;
- ledit photodétecteur est un photodétecteur analogique dont le signal analogique fournit une mesure représentative de l'énergie des photons émis par ledit scintillateur monolithique au cours du temps ;
- ledit dispositif d'acquisition est configuré pour la détection et le filtrage des événements de scintillation grâce à un algorithme lui permettant de mesurer un premier temps auquel ledit signal analogique dépasse un premier seuil représentatif du premier photon détecté et de mesurer un second temps auquel ledit signal analogique dépasse un second seuil représentatif d'un nombre n de photons détectés, puis de calculer le délai qui sépare le premier et le second temps.

Selon une autre particularité, ledit dispositif d'acquisition recueille le signal du scintillateur sur une pluralité de pixels et compare le temps du premier photon détecté sur chacun des i pixels, afin de déterminer la valeur minimum de ce temps sur l'ensemble des pixels et d'estimer ainsi le temps d'arrivée du premier photon émis par le scintillateur.

Selon une autre particularité, ledit dispositif d'acquisition calcule une pente de montée du signal entre les deux seuils et élimine les événements détectés dont la pente de montée est inférieure à une valeur déterminée, afin d'éliminer le bruit du signal et ne conserver que des événements de scintillation validés.

Selon une autre particularité, ladite unité de traitement établit une carte du temps d'arrivée des photons à partir des événements filtrés par ledit dispositif d'acquisition pour fournir une première estimation de la position d'un événement de scintillation et d'un disque de photons non diffusés, puis compare au moins un modèle de loi d'émission des photons par le scintillateur avec le nombre de photons détectés dans ce disque à différents temps après l'arrivée du premier photon, afin de corriger en conséquence le diamètre de ce disque et donc d'améliorer la précision de la mesure de la profondeur de l'événement.

Selon une autre particularité, l'unité de traitement corrige le retard probable entre les valeurs mesurées et réelles du temps d'arrivée du premier photon, en comparant ces valeurs mesurées avec au moins un modèle de lois d'émissions des photons par le scintillateur, de préférence en tenant compte des mesures des temps d'arrivée du premier et un nième photon dans les pixels adjacents.

### DESCRIPTION DES FIGURES ILLUSTRATIVES

D'autres particularités et avantages de la présente invention apparaîtront plus clairement à la lecture de la description ci-après, faite en référence aux dessins annexés, dans lesquels :
- la figure 1 représente une vue en perspective d'un système d'imagerie selon certains modes de réalisation ;
- la figure 2 représente une vue en perspective d'un système d'imagerie selon d'autres modes de réalisation ;
- les figures 3A, 3B, 3C et 3D représentent des vues schématiques en transparence de la composition de divers modules de détection qui peuvent équiper des systèmes d'imagerie selon divers modes de réalisation, tandis que les figures 3E et 3F représentent des courbes de détection de photons, avec le temps en abscisses et l'énergie en ordonnées ;
- la figure 4 représente une vue en perspective d'un système d'imagerie selon certains modes de réalisation ;
- la figure 5 représente une vue en perspective d'un système d'imagerie selon d'autres modes de réalisation ;

### DESCRIPTION DES MODES DE REALISATION PREFERES DE L'INVENTION

La présente invention concerne un système et un procédé d'imagerie par détection de rayonnement gamma comprenant au moins une unité (5) de traitement analysant au moins un signal fourni par au moins un ensemble de modules (CP, P) de détection montés sur un châssis (1) et comportant, d'une part, au moins un module de type caméra Compton possédant un champ de visée (CV) dirigé vers un volume délimité par le châssis et, d'autre part, au moins un couple de modules PET de détection de coïncidences, diamétralement opposés l'un par rapport à l'autre sur ledit châssis et définissant un axe (A) d'imagerie, ladite unité (5) de traitement analysant le signal issu dudit module de type Compton pour déterminer l'intersection dudit axe (A) d'imagerie avec ledit champ de visée (CV) et déterminer les orientations et/ou emplacements optimaux des divers modules (CP, P) de détection sur le châssis pour que l'axe (A) d'imagerie passe par la source dudit rayonnement gamma dans ledit objet (O) à imager. Ainsi, on comprend que l'on bénéficie du champ de visée (CV) large de la caméra Compton (environ 45° d'ouverture angulaire en général) pour localiser la source du rayonnement gamma (avec une précision angulaire de l'ordre de 5° le plus souvent) et l'on utilise cette information pour positionner les modules à utiliser en mode PET pour faire l'acquisition d'une image plus précise (dans un axe de visée / d'imagerie plus réduit). De façon connue en soit, chacun des modules de détection comprend au moins un scintillateur (2) émettant des photons lorsqu'il est soumis à l'influence dudit rayonnement gamma, au moins un photodétecteur (3) générant ledit signal (S) en réponse auxdits photons émis par ledit scintillateur (2) et au moins un dispositif d'acquisition (4) qui recueille ledit signal pour le transmettre à ladite unité (5) de traitement. Le module de type Compton comporte de préférence un scintillateur (2) qui comprend au moins une plaque de cristal scintillateur (P1), dit rapide, dont le temps de montée au pic de lumière est inférieur à 1ns, de sorte que ce module est apte à produire une image en utilisant la diffusion Compton. De façon plus spécifique, ledit châssis (1) est ouvert, sur au moins un coté entre les deux calottes (10), de sorte à laisser l'accès libre audit objet (O) à imager dans ledit volume.

Dans certains modes de réalisation, ledit châssis (1) comprend une pluralité d'emplacements répartis selon au moins une portion de deux calottes sphériques (10) en vis à vis l'une de l'autre et définissant un volume d'imagerie à l'intérieur du système, lesdites calottes (10) étant séparées d'une distance (D) permettant d'accueillir un objet (O) à imager dans ledit volume. Les modules PET du couple de modules de détection de coïncidences sont donc montés diamétralement opposés l'un par rapport à l'autre sur ledit châssis, chacun sur l'une des deux calottes (10).

Ainsi, on comprend donc que le présent système utilise la caméra Compton pour repérer la zone d'intérêt (source de rayonnement gamma) dans l'objet (O) à imager et que l'unité de traitement détermine la position des modules PET permettant que l'axe d'imagerie (A) passe par cette zone, afin de réaliser une image précise, en Mode PET (détection de coïncidences) à deux dimensions de cette zone d'intérêt. On comprend donc que le châssis définit un volume entre deux calottes (ou arcs de cercles, voire arcs elliptiques) et présente une ouverture sur au moins un côté du châssis par laquelle l'objet reste accessible (et peut être introduit dans ledit volume) et que, à l'intérieur de ce volume, il est possible de restreindre le champ couvert par les modules de détection. Un tel système peut avantageusement être utilisé pour réaliser de l'imagerie préclinique ou pour de l'imagerie clinique d'un organe spécifique ou même pour réaliser des biopsies guidées par imagerie. Un des objectifs visés par les objets de la présente demande concerne également le fait de pouvoir réaliser des images localement précises d'un objet (ou sujet) volumineux, tel qu'un humain ou un gros animal (porc, cheval par exemple) et l'imagerie réalisée via les caméras Compton (C, CP) donne une image présentant un champ de vue large et une résolution modérée. Les zones d'intérêt sont ensuite détaillées en déplaçant les détecteurs de coïncidence pour obtenir une image précise au sein de leur champ de vue plus limité en taille.

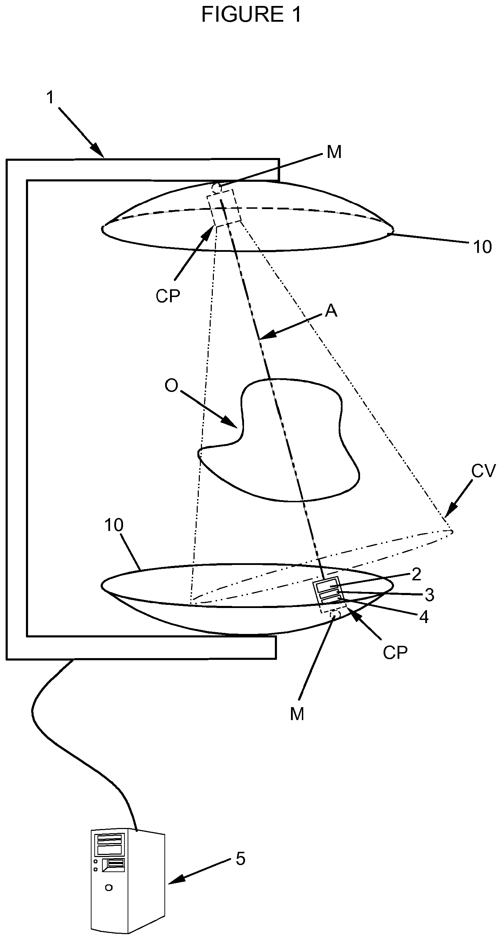

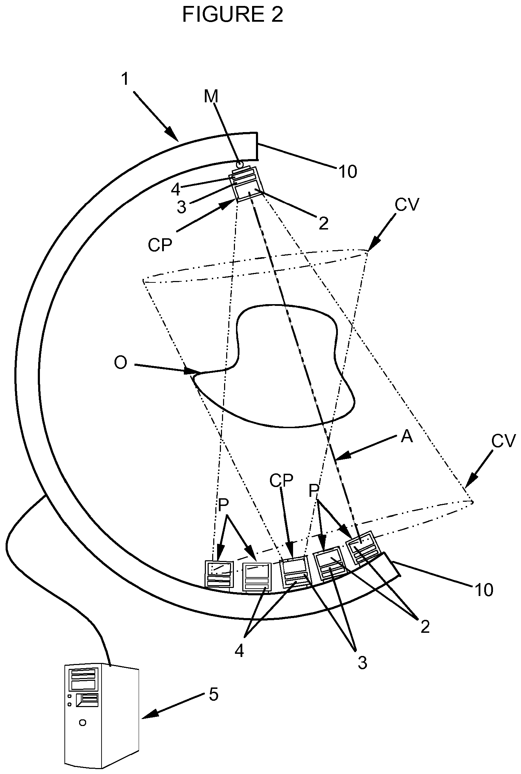

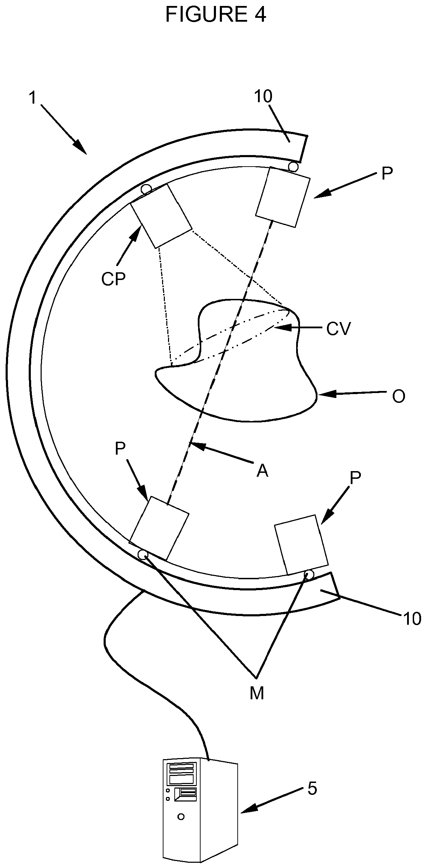

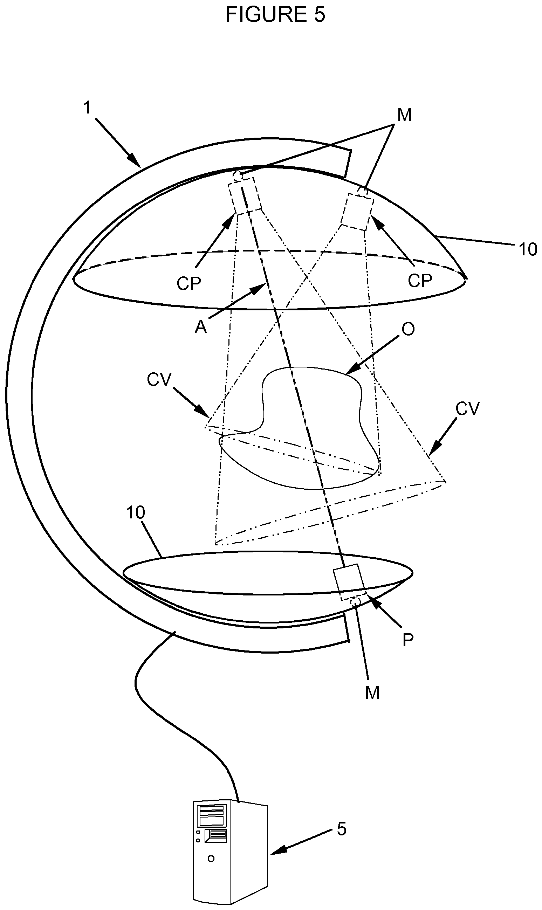

Le terme "sphérique" désigne en fait dans la présente demande une disposition géométrique qui peut être légèrement elliptique, du moment qu'il est possible de disposer des modules en vis-à-vis l'un de l'autre, avec un axe de symétrie commun adapté à une détection de coïncidence en mode PET. L'expression "au moins une portion de calotte sphérique", concernant la répartition des emplacements des modules, couvre bien entendu des définitions telles que des calottes disposées à deux pôles opposés d'une boule (e.g., sphérique ou elliptique) ou des calottes légèrement excentrées mais possédant, deux à deux, des emplacements diamétralement opposés ou au moins en vis-à-vis l'un de l'autre. De plus, cette expression "au moins une portion de calotte sphérique" couvre non seulement des calottes sphériques opposées et identiques, par exemple comme illustré sur la figure 1, ou des calottes opposées mais de tailles différentes, par exemple comme illustré sur la figure 5, mais elle couvre en fait également (par le mot « portion ») dans la présente demande des répartitions des emplacements seulement selon des arcs (de cercles ou elliptiques) en vis-à-vis l'un de l'autre, par exemple comme représenté sur la figure 2 ou la figure 4. En effet, le châssis (1) peut en fait couvrir un seul plan ou plusieurs plans et il peut par exemple se limiter à une forme de C ou même définir seulement deux arcs définissant lesdits emplacements et maintenus séparés l'uns de l'autre par toute structure respectant ladite distance (D) et prévoyant une ouverture qui offre ledit accès libre audit objet (O) à imager. Ainsi, divers modes de réalisation prévoient une large gamme de produits partant de systèmes très simples, légers et économiques en sensiblement seulement 2 dimensions, jusqu'à des systèmes plus complexes couvrant 3 dimensions et facilitant la mise en place de l'imagerie grâce à leur configuration complète, avantageusement pilotée par ladite unité de traitement. Le terme "événements" relatif aux modules de détection est parfaitement connu de l'homme de métier et désigne l'interaction d'un rayonnement ionisant avec un matériau dit "scintillant" ou "scintillateur" qui émet au moins un photon lorsqu'une particule entre en collision avec l'un des atomes qui le constitue. Les termes "diffusion Compton" ou "caméra Compton" sont connus de l'homme de métier et concernent bien entendu la détection de deux évènements qui sont très proches dans le temps et sont détectés par un seul scintillateur ou deux scintillateurs proches l'un de l'autre, comme détaillé par exemple dans la demande WO2016185123. Les termes "module PET" ou "en mode PET" ou "détection de coïncidence" sont également connus de l'homme de métier et désignent bien entendu le fait que l'on mesure le temps séparant des événements qui se produisent entre deux scintillateurs diamétralement opposés autour de la source de rayonnement gamma.

Dans certains modes de réalisation, le système comporte au moins un moteur (M), piloté par ladite unité de traitement (5) et contrôlant la mobilité d'au moins un desdits modules (CP, P) de détection sur ledit châssis et/ou de l'objet à observer à l'intérieur dudit volume pour obtenir lesdites orientations et/ou lesdits emplacements optimaux définis par ladite unité de traitement (5). On comprend donc que divers modes de réalisation prévoient au moins un moteur pour pouvoir déplacer l'objet à imager (O) selon les positions des modules de détection déterminées par l'unité de traitement et/ou au moins un moteur (M) permettant également de contrôler l'orientation relative et/ou le déplacement relatif des modules, afin d'aligner l'axe d'imagerie sur la zone d'intérêt, à l'intérieur d'au moins une partie dudit volume. Ainsi, en déplaçant et/ou en orientant au moins un module ou plusieurs modules, l'unité de traitement permet que les modules soient alignés sur une zone d'intérêt, de préférence ciblée au préalable par un module Compton ou hybride. Divers modes de réalisation prévoient donc des moyens de déplacement de l'objet dans au moins une portion du volume à l'intérieur du châssis et/ou des moyens de déplacement d'au moins un des modules sur le châssis. Le terme "moteur" est utilisé dans la présente demande selon son acception générale désignant en fait la fonction de contrôler un mouvement, quel que soit le type de mouvement concerné, ce qui implique éventuellement un pivotement autour d'un axe pour définir une orientation, aussi bien qu'un déplacement dans deux ou trois dimensions, mais également une combinaison de ces deux types de mouvements. L'homme de métier en appréciera plus facilement la portée à la lecture des définitions fournies ci-après et des divers modes de réalisation illustratifs qui en découlent.

Dans certains modes de réalisation, au moins un desdits ensembles de modules (P, CP) de détection comporte en fait seulement deux modules, grâce à un premier module (CP), dit "hybride", dont le scintillateur (2) comprend au moins une plaque de cristal scintillateur (P1), dit rapide, dont le temps de montée au pic de lumière est inférieur à 1ns, ledit module "hybride" étant apte à produire à la fois une diffusion Compton et une absorption d'au moins une partie du rayonnement gamma pour une détection de coïncidence entre les événements dans ce premier module hybride (CP) et les événements dans un second module (CP, P) de détection avec lequel ce premier module hybride (CP) forme donc ledit couple de modules PET de détection de coïncidences. On comprend donc que, dans certains modes de réalisation, le système tire avantage de la possibilité d'avoir un module hybride formant à la fois la caméra Compton et un des modules PET du couple de détecteurs de coïncidences. Ainsi, au lieu d'avoir trois modules au sein de chaque ensemble, il est possible de n'en avoir que deux (dont un hybride) ou d'avoir des ensembles à trois modules et des ensembles à deux modules. De plus, il est bien entendu possible d'avoir au moins un premier ensemble "mixte" Compton-PET (à trois modules ou à deux modules, dont un hybride), combiné à au moins un autre ensemble ne comportant que deux modules PET de détection de coïncidences et bénéficiant donc du module Compton du premier ensemble.

Dans certains modes de réalisation, ledit second module (CP, P) de détection de chaque ensemble est soit un module hybride (CP) également, soit un module (P), dit "PET", apte à produire seulement une absorption photoélectrique pour une détection de coïncidence d'événements par rapport à l'autre module de détection avec lequel il forme ledit couple de modules PET de détection de coïncidences. Des exemples de ces variantes sont montrées sur les figures 1, 2, 4 et 5. Dans certains modes de réalisation, chaque ensemble comporte plusieurs seconds modules (CP, P) qui peuvent être fixes (i.e., immobiles) et qui sont disposés en vis-à-vis du premier module hybride (CP) qui est orientable par ledit moteur (M) pour aligner l'axe (A) d'imagerie entre ledit module hybride (CP) et l'un ou l'autre de ces seconds modules (CP, P). Un exemple de ce type de configuration est représenté sur la figure 2. Ce type de configuration a pour avantage de limiter le coût des moyens moteurs (M) à utiliser, mais oblige souvent à augmenter le nombre de modules de détection. Dans certains modes de réalisation, ledit moteur (M) est apte à déplacer au moins ledit second module (CP, P) sur ledit châssis (1), pour le placer en vis-à-vis du premier module hybride (CP) qui est orienté par ledit moteur (M) pour aligner l'axe (A) d'imagerie entre le premier et le second module. Dans certains modes de réalisation, le premier et le second module sont tous les deux des modules hybrides (CP) dont au moins un d'entre eux est orientable et au moins un d'entre eux est déplaçable sur le châssis (1), par ledit moteur (M). Ainsi, le module hybride déplaçable (muni d'un moteur de déplacement) pourra être placé en face du module hybride orientable pour que l'axe d'imagerie PET soit optimal d'après l'imagerie Compton dans le champ de visée (CV). On notera que, lorsqu'il est prévu plusieurs modules en vis-à-vis d'un module hybride, comme par exemple représenté sur la figure 2, il n'est forcément pas nécessaire que le second module hybride soit déplaçable puisque l'axe d'imagerie (A) en PET pourra être déterminé avec l'un des autres modules. De plus, l'utilisation de deux modules Compton (qu'ils soient hybrides ou non et dans quelque configuration que ce soit, comme par exemple sur les figures 1, 2 ou 5) permet une triangulation pour estimer la profondeur de la source de rayonnement, comme détaillé ci-après.

Dans certains modes de réalisation, ladite unité (5) de traitement du signal analyse les signaux issus de chaque ensemble de modules (CP, P) de détection et détermine à la fois les événements de diffusion Compton et les événements d'absorption photoélectrique, pour obtenir la position tridimensionnelle, l'énergie et la séquence temporelle des interactions, Compton et photoélectriques, du rayonnement gamma dans le volume entre les modules (CP, P) de détection de chaque ensemble. Dans certains modes de réalisation, l'unité (5) de traitement du signal mesure, dans le signal issu du module hybride (CP), un seuil temporel de coïncidence dans une fenêtre temporelle inférieure au temps de transfert maximum de la lumière dans le module hybride, à l'intérieur de la plaque unique (P1) ou entre les plaques (P1) et (P2), pour identifier des événements Compton valides. Une telle utilisation a déjà été décrite dans les demandes WO2016185123 et WO2017077164 et il n'est donc pas nécessaire de la décrire plus en détail.

Dans certains modes de réalisation, l'unité de traitement calcule le temps de vol des photons entre les deux modules du couple de module PET premier module hybride (CP) et le second module (CP, P) pour mesurer la profondeur de la source de rayonnement gamma dans l'objet (O) à imager selon l'axe d'imagerie. Ce calcul du temps de vol (généralement appelé "Time Of flight" dans le domaine) permet en effet de mesurer la profondeur de la source du rayonnement gamma, comme par exemple décrit dans l'article scientifique "Time-of-Flight PET" By Joel S. Karp, PET center of excellence newsletter, Volume 3, Issue 4 FALL 2006 ; SNM advancing molecular imaging & therapy (dans les 4 premiers paragraphes, en particulier). Ce temps de vol peut être calculé entre deux modules d'un couple de modules en mode PET, quel que soit le type de modules de ce couple, qu'il s'agisse d'un premier module hybride (CP) et un second module (P) PET ou bien de deux modules (P) PET ou même deux modules hybrides (CP). Dans certains modes de réalisation, l'unité de traitement utilise le signal d'au moins un couple module de type caméra Compton pour combiner leurs imageries Compton et réaliser une triangulation afin de mesurer la profondeur de la source de rayonnement gamma dans l'objet (O) à imager. Ainsi, dans les modes de réalisation dans lesquels ledit ensemble de modules de détection comporte deux modules PET et au moins un module Compton, comme par exemple dans la figure 4, le module Compton permet une triangulation en combinaison avec l'axe d'imagerie du couple de modules PET pour une première estimation de la profondeur, mais le couple de modules PET permet de préciser la profondeur grâce au « Time OF Flight ». De plus, dans certains modes de réalisation, il est prévu d'avoir deux modules Compton, par exemple situés du même côté de l'objet (O), notamment avec deux modules hybrides comme dans l'exemple représenté sur la figure 5 ou avec un module Compton à côté d'un module hybride et en face d'un module PET ou Compton selon diverses variantes de réalisation. Dans toutes ces diverses variantes de réalisation à plusieurs modules Compton, l'unité de traitement (5) sera donc capable d'utiliser les signaux d'imagerie Compton pour réaliser une triangulation afin de mesurer la profondeur. Avantageusement, en combinant au moins un couple de modules Compton avec au moins un couple de modules PET, il est possible d'affiner la mesure de la profondeur. On comprend des diverses variantes décrites ici que ces couples de modules Compton et PET peuvent en fait comporter un module en commun (grâce à au moins un module hybride). De plus, dans le cas où l'on utilise deux modules hybrides, cette combinaison entre la mesure du Time Of Flight et la triangulation Compton peut avantageusement être réalisée à partir des signaux du même couple de modules hybrides, comme par exemple ceux de la figure 1, mais de préférence après déplacement du couple de modules hybrides afin combiner de façon optimale leurs signaux en mode PET et leurs signaux en mode Compton.

Dans certains modes de réalisation, l'unité de traitement du signal réalise une imagerie Compton à partir d'événements dont l'énergie n'est pas de 511keV (notamment pour compenser le fait que l'imagerie PET ne fournit pas suffisamment d'informations pour estimer la profondeur de la source du rayonnement et détecte uniquement des événements de 511keV en coïncidence sur deux modules diamétralement opposés. De plus, dans certains modes de réalisation, il est possible de réaliser des injections de plusieurs radios-éléments différents pour obtenir une imagerie différentielle et/ou une injection d'un radioélément générant des événements d'au moins deux niveaux d'énergie différents (comme par exemple le Na22 qui génère des événements de 511 keV et des événements de 1.3 MeV).

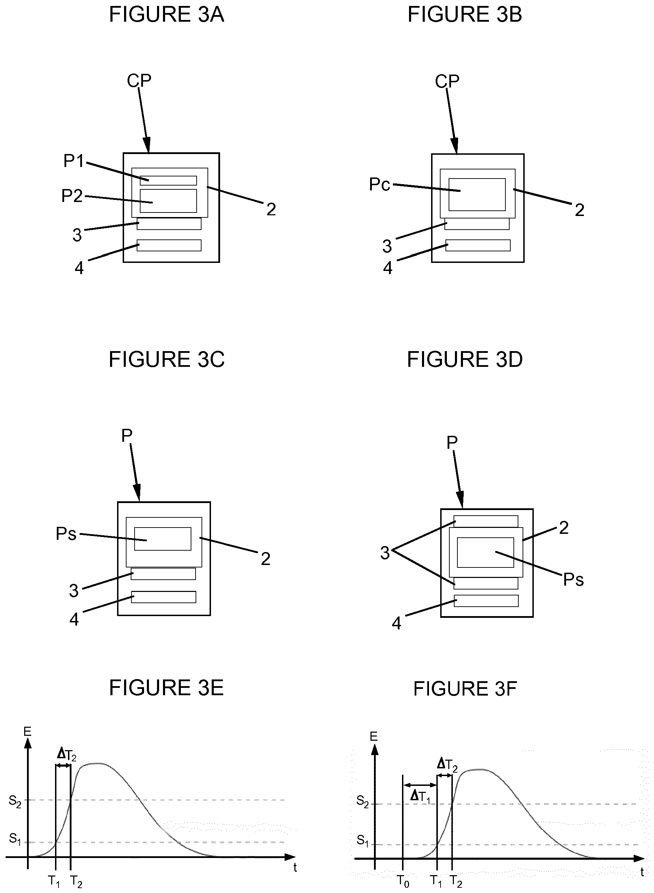

Dans certains modes de réalisation, le module hybride (CP) comporte une seule plaque de cristal scintillateur (Pc, figure 3B) présentant une épaisseur supérieure ou égale au libre parcours moyen du rayon gamma dans le cristal considéré, de préférence supérieure à 5 mm. Un tel module hybride est connu de l'art antérieur et notamment de la demande WO2016185123. Dans d'autres modes de réalisation, comme par exemple représenté sur la figure 3A, le module hybride (CP) comporte une première plaque de cristal scintillateur (P1) présentant une épaisseur inférieure au libre parcours moyen du rayon gamma dans le cristal considéré et une seconde plaque de cristal scintillateur (P2) présentant une épaisseur supérieure ou égale au libre parcours moyen du rayon gamma dans le cristal considéré et permettant d'absorber au moins 50% de l'énergie du rayonnement gamma, ladite seconde plaque (P2) étant séparée de la plaque P1 par une distance d'au moins 10 mm, de préférence supérieure à l'épaisseur de la plaque la plus épaisse. Dans certains modes de réalisation, le couple de modules diamétralement opposés est formé par deux modules hybrides possédant ces deux types de plaques. De tels modes de réalisation présentent l'avantage de permettre une mesure temporelle très précise entre deux plaques fines opposées, par exemple comme décrit dans la demande WO2017077164. En effet, dans un système qui comporte deux modules hybrides en vis à vis l'un de l'autre et où ces modules hybrides comportent une plaque mince P1 de diffuseur et une plaque épaisse P2 d'absorbeur, l'unité de traitement bénéficie d'une résolution temporelle (CRT) améliorée en mesurant mesure les temps de vols précis grâce aux événements en coïncidence entre les 2 plaques minces P1 de Diffuseur. De même, dans certains modes de réalisation, comme par exemple représenté sur la figure 3D, au moins une partie des modules comportent un photodétecteur (3) sur chacune des deux faces opposées de chaque scintillateur (2), ce qui permet d'améliorer la résolution temporelle et facilite notamment la mesure de la profondeur avec l'imagerie PET photoélectrique. Dans certains de ces modes de réalisation où les modules hybrides comportent deux plaques, la première plaque (P1) est de formule chimique du type ALnX3 : Ce, A étant tout type d'élément alcalin, de préférence d'un type proche du Césium, Ln étant un Lanthanide tel que le Lanthane ou le Cérium, X étant un Fluorure tel que le Chlore, le Fluore ou le Brome.

Dans certains modes de réalisation, par exemple comme représenté sur la figure 3C, au moins un module dudit couple est un module "simple" (P) apte uniquement à une imagerie de type PET par détection de coïncidence. Un tel module comporte alors généralement une plaque de cristal scintillateur (Ps) présentant une épaisseur supérieure ou égale au libre parcours moyen du rayon gamma dans le cristal considéré et permettant d'absorber au moins 50% de l'énergie du rayonnement gamma. On notera d'ailleurs que cette plaque (Ps) pourra être sensiblement du même type que la deuxième plaque (P2) dans les scintillateurs (2) des modules hybrides (CP) à deux plaques.

La présente demande peut également concerner divers modes de réalisation d'un système et/ou d'un procédé utilisant au moins un « double seuil ». Ces divers modes de réalisation sont indépendants mais non exclusifs des modes de réalisation dans lesquels le système utilise un châssis tel que décrit dans la présente demande et/ou une combinaison de détecteurs de type PET et de type Compton. Dans de tels modes de réalisation, on tire avantage de l'utilisation, par l'unité de traitement, d'un double seuil sur la mesure du temps auquel une certaine quantité d'énergie/de photons a été détectée sur un pixel pour pallier à certains inconvénients de l'art antérieur, notamment en ce qui concerne les cristaux monolithiques et les photodétecteurs analogiques, par exemple comme expliqué dans le préambule de la présente demande. Ainsi, divers modes de réalisation concernent un système d'imagerie par détection de rayonnement gamma comprenant au moins une unité (5) de traitement analysant au moins un signal fourni par au moins un ensemble de modules (CP, P) de détection montés sur au moins un châssis (1), chacun des modules comprenant au moins un scintillateur (2) émettant des photons, lorsqu'il est soumis à l'influence dudit rayonnement gamma, et au moins un photodétecteur (3) générant ledit signal (S) en réponse auxdits photons émis par ledit scintillateur (2), le système étant caractérisé en ce que
- ledit scintillateur (2) est un cristal scintillateur monolithique ;
- ledit photodétecteur (3) est un photodétecteur (3) analogique dont le signal analogique fournit une mesure représentative des photons détectés (i.e., nombre et/ou énergie) par ledit scintillateur (2) monolithique au cours du temps ;
- ledit dispositif d'acquisition (4) est configuré pour la détection et le filtrage des événements de scintillation grâce à un algorithme lui permettant de mesurer un premier temps (T1) auquel ledit signal analogique dépasse un premier seuil (S1) représentatif du premier photon détecté et de mesurer un second temps (T2) auquel ledit signal analogique dépasse un second seuil (S2) représentatif d'un nombre n de photons détectés, puis de calculer le délai (ΔT2) qui sépare le premier (T1) et le second (T2) temps.

En pratique, le photodétecteur comporte en fait une pluralité (i) de pixels qui fournissent tous un signal analogique que le dispositif d'acquisition (4) transmet à l'unité de traitement (5). On comprend donc que le dispositif d'acquisition (4) réalise en fait un comptage et le calcul de filtrage sur chacun des (i) pixels.

De préférence, ce pré-traitement de calcul des délais permettant la détection et le filtrage des événements doit être fait "à la volée' par le dispositif d'acquisition (4), par exemple implémenté sous la forme d'un ASIC ou d'un FPGA, car les délais sont très rapides (par exemple inférieur à 10ns) et ce filtrage serait très probablement inefficace s'il devait être réalisé par l'unité de traitement (5) qui peut, par exemple, être un PC ou un serveur distant. On comprend de ce qui précède que la présente demande propose de mesurer les temps auxquels le signal dépasse un premier seuil pour tenter de déterminer le temps d'arrivée du premier photon, puis un second seuil pour déterminer le temps auquel le signal aura atteint une valeur suffisamment significative pour être réellement représentatif d'un événement de scintillation et non du bruit (e.g., électronique).

Il est connu dans l'art antérieur des méthodes de filtrage du signal utilisant un seuil de détection, comme par exemple dans l'article de Frach et al. "The Digital Silicon Photomultiplier - Principle of Opération and Intrinsic Detector Performance" IEEE Nuclear Science Symposium Conférence Record (pp. 1959-1965) 2009, N28-5. Cependant, dans ces méthodes, il est généralement fait usage d'un calcul de l'intégrale du signal sur une durée fixe, afin de déterminer simplement si le signal dépasse une énergie déterminée au cours de cette durée, sans se préoccuper du temps exact ni de la forme du signal. Dans la présente demande, au contraire, il est proposé d'utiliser l'énergie instantanée et de déterminer le temps auquel cette énergie dépasse un seuil. Grâce aux deux seuils (S1, S2), la présente demande permet de calculer la pente du signal, ce qui permet de comparer le signal recueilli à des modèles d'émission connus pour les cristaux scintillateurs et à des simulations de la répartition spatiale des photons en utilisant ces modèles.

Dans certains modes de réalisation, le dispositif d'acquisition (4) recueille le signal du scintillateur (2) sur une pluralité (i) de pixels et compare le temps (T1) du premier photon détecté sur chacun des i pixels, afin de déterminer la valeur minimum de ce temps sur l'ensemble des pixels et d'estimer ainsi le temps (T0) d'arrivée du premier photon émis par un événement dans le scintillateur. On comprend que le dispositif d'acquisition (4) mesure donc, pour chacun des i pixels la différence (ΔT1) entre ce temps (T1) de ce pixel et la valeur minimum de ce temps (T1) parmi tous les pixels, ce qui permet de trouver le pixel dans lequel ce temps est le plus petit et donc estimer le temps (T0) d'arrivée du premier photon émis.

Dans certains modes de réalisation, ledit dispositif d'acquisition (4) calcule une pente de montée du signal entre les deux seuils et élimine les événements détectés dont la pente de montée est inférieure à une valeur déterminée, afin d'éliminer le bruit du signal et ne conserver que des événements de scintillation validés.

Dans certains modes de réalisation, ladite unité de traitement (5) établit une carte du temps d'arrivée des photons à partir des événements filtrés par ledit dispositif d'acquisition (4) pour fournir une première estimation de la position d'un événement de scintillation et d'un disque de photons non diffusés, puis compare au moins un modèle de loi d'émission des photons par le scintillateur (2) avec le nombre de photons détectés dans ce disque à différents temps après l'arrivée du premier photon, afin de corriger en conséquence le diamètre de ce disque et donc d'améliorer la précision de la mesure de la profondeur de l'événement.

Dans certains modes de réalisation, l'unité de traitement corrige le retard probable entre les valeurs mesurées et réelles du temps (T0) d'arrivée du premier photon émis, en comparant ces valeurs mesurées avec au moins un modèle de lois d'émissions des photons par le scintillateur (2), de préférence en tenant compte des mesures des temps d'arrivée du premier et un nième photon dans les pixels adjacents. Cette comparaison peut s'effectuer soit en comparant T0 avec les T1 de tous les pixels, soit en comparant T0 et T2 sur le pixel le plus rapide. On notera que l'on réfère ici à un dispositif d'acquisition de signal qui réalise un pré-traitement des événements détectés afin d'obtenir des événements validés / filtrés, puis à une unité de traitement (5) qui réalise des calculs plus complexes. En pratique, le dispositif d'acquisition (4) pourra être un Asic ou un FPGA simple pour calculer les seuils et filtrés les événements, tandis que l'unité de traitement (5) vectorise les signaux et nécessite des capacités de calculs plus importantes, comme par exemple un FPGA de taille importante et/ou des moyens informatiques (tels qu'un ordinateur, un serveur ou éventuellement tout autre appareil muni de ressources de calcul suffisantes).

Le modèle de la loi d'émission est basé sur la connaissance des courbes d'émission des divers types de cristaux scintillateurs utilisables dans la présente invention et dont au moins certains sont décrits dans la présente demande. De telles courbes ont notamment été décrites dans l'article de Seifert et al 2012 JINST 7 P09004

Ainsi, certains modes de réalisation de l'invention peuvent concerner également un procédé d'imagerie par détection de rayonnement gamma au sein d'un système comprenant au moins une unité (5) de traitement analysant au moins un signal fourni par au moins un ensemble de modules (CP, P) de détection montés sur au moins un châssis (1), chacun des modules comprenant au moins un cristal scintillateur (2) monolithique émettant des photons, lorsqu'il est soumis à l'influence dudit rayonnement gamma, au moins un photodétecteur (3) analogique dont le signal analogique fournit une mesure représentative de l'énergie des photons émis par ledit scintillateur (2) monolithique au cours du temps et au moins un dispositif d'acquisition (4) recueillant ledit signal pour le transmettre à ladite unité de traitement (5), le procédé étant caractérisé en ce qu'il comporte :
- la mesure d'un premier temps (T1) auquel ledit signal analogique dépasse un premier seuil (S1) représentatif du premier photon détecté ;
- la mesure d'un second temps (T2) auquel ledit signal analogique dépasse un second seuil (S2) représentatif d'un nombre n de photons détectés ;
- le calcul du délai (ΔT2) qui sépare le premier (T1) et le second (T2) temps.

Dans certains modes de réalisation, le procédé comporte une collecte du signal du scintillateur (2), par le dispositif d'acquisition (4), sur une pluralité (i) de pixels puis une comparaison (ΔT1) entre le temps (T1) du premier photon détecté sur chacun des i pixels, afin de déterminer la valeur minimum de ce temps sur l'ensemble des pixels et d'estimer ainsi le temps (T0) d'arrivée du premier photon émis par le scintillateur.

Dans certains modes de réalisation, le procédé comporte un calcul, par ledit dispositif d'acquisition (4), d'une pente de montée du signal entre les deux seuils et élimine les événements détectés dont la pente de montée est inférieure à une valeur déterminée, afin d'éliminer le bruit du signal et ne conserver que des événements de scintillation validés.

Dans certains modes de réalisation, le procédé comporte un établissement, ladite unité de traitement (5), d'une carte du temps d'arrivée des photons à partir des événements filtrés par ledit dispositif d'acquisition (4) pour fournir une première estimation de la position d'un événement de scintillation et d'un disque de photons non diffusés, puis une comparaison d'au moins un modèle de loi d'émission des photons par le scintillateur (2) avec le nombre de photons détectés dans ce disque à différents temps après l'arrivée du premier photon, afin de corriger en conséquence le diamètre de ce disque et donc d'améliorer la précision de la mesure de la profondeur de l'événement.

Dans certains modes de réalisation, le procédé comporte une correction, par ladite unité de traitement (5), l'unité de traitement corrige le retard probable entre les valeurs mesurées et réelles du temps d'arrivée du premier photon (T0), en comparant ces valeurs mesurées avec au moins un modèle de lois d'émissions des photons par le scintillateur (2), de préférence en tenant compte des mesures des temps d'arrivée du premier et un nième photon dans les pixels adjacents.

On notera que l'on peut aussi décrire la méthode de la manière suivante, sans que cela ne doive être interprété comme limitant la portée de la demande. Le premier seuil par pixel (S1) est réglé le plus bas possible 1 à 2 photoélectrons. Ce seuil sert à mesurer le CRT et à mesurer le temps relatif d'allumage des pixels afin d'identifier le cercle des non-diffusés et les interactions Compton. Ce seuil bas impose un niveau de bruit élevé qui doit être filtré. Seul le temps précis du premier pixel (T0) détecté doit être connu avec précision. Seule la différence (ΔT1) de temps d'allumage avec les autres pixels nous intéresse (T1-T0). Le deuxième seuil (S2) sert essentiellement à estimer la densité de photons initiale par pixels et au filtrage des événements pertinents. On utilisera ΔT2 (T1-T2) pour la reconstruction. Il sert également au filtrage des événements. Ce second seuil doit être réglable probablement entre 5 et 20 photoélectrons réglable et en fonction du type de cristal) et de l'énergie des interactions auxquelles on s'intéresse. L'étendue de réglage doit être importante car elle permet de capter ou d'éteindre les zones peu denses de la distribution de lumière. Le délai pour le passage du seuil doit lui aussi être réglable : Au plus court 1 ns, de préférence 2,5 ns et au plus long 50 ns, de préférence 20ns. Le temps d'intégration de la lumière pour calculer l'énergie doit être réglable. Aujourd'hui par exemple pour CeBr3 ont pourrait envisager 50 ns, 100 ns ou 200 ns.

Filtrage des événements :
On applique un seuil temporel de délai de validation réglable au niveau de chaque pixel
Si ΔT2 (T2-T1) < seuil = événement accepté
Si ΔT2 (T2-T1) > seuil = événement rejeté
La valeur du délai minimal doit être <50ns (2,5 ns étant une valeur idéale, mais il est préférable d'avoir plusieurs valeurs possibles)
Au niveau du deuxième seuil, il faut une validation d'événement. Un événement est valide si n pixels ont passé le seuil, tel que défini ci-dessus.
(n = 4 à 60... réglable et en fonction du type de cristal).

Le délai entre T0 (premier pixel détecté) et T1 (passage du seuil à 1 Phe sur la tuile i) correspond à la distribution de temps de l'événement.

Le délai entre T1 et T2 sert de seuil pour accepter/rejeter l'événement et également à améliorer la précision temporelle de la reconstruction du temps et du cercle des non-diffusés (post processing).

La présente demande décrit diverses caractéristiques techniques et avantages en référence aux figures et/ou à divers modes de réalisation.

L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné. Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Système d'imagerie par détection de rayonnement gamma comprenant au moins une unité (5) de traitement, au moins un châssis (1), et au moins un ensemble de modules (CP, P) de détection montés sur ledit au moins un châssis (1) et fournissant au moins un signal analysé par ladite au moins une unité (5) de traitement, chacun des modules comprenant au moins un scintillateur (2) émettant des photons, lorsqu'il est soumis à l'influence dudit rayonnement gamma, au moins un photo-détecteur (3) générant ledit signal (S) en réponse auxdits photons émis par ledit scintillateur (2) et au moins un dispositif d'acquisition (4) qui recueille ledit signal pour le transmettre à ladite unité (5) de traitement, le système étant **caractérisé en ce que**:
- ledit châssis (1) comprend une pluralité d'emplacements répartis selon au moins une portion de deux calottes sphériques (10) en vis à vis l'une de l'autre et définissant un volume d'imagerie à l'intérieur du système, lesdites calottes (10) étant séparées d'une distance (D) permettant d'accueillir un objet (O) à imager dans ledit volume ;
- ledit châssis (1) étant ouvert, sur au moins un coté entre les deux calottes (10), de sorte à laisser l'accès libre audit objet (O) à imager dans ledit volume ;
- ledit ensemble de modules (CP, P) de détection comporte au moins un module de type caméra Compton, possédant un champ de visée (CV) dirigé entre les deux calottes (10) et dont le scintillateur (2) comprend au moins une plaque de cristal scintillateur (P1), dit rapide, dont le temps de montée au pic de lumière est inférieur à 1ns, de sorte que ce module est apte à produire une image en utilisant la diffusion Compton ;
- ledit ensemble de modules (CP, P) de détection comporte au moins un couple de modules PET de détection de coïncidences, diamétralement opposés l'un par rapport à l'autre sur ledit châssis, chacun sur l'une des deux calottes (10), et définissant un axe (A) d'imagerie ;
- ladite unité (5) de traitement analyse le signal issu dudit module de type Compton pour déterminer l'intersection dudit axe (A) d'imagerie avec ledit champ de visée (CV) et déterminer les orientations et/ou emplacements optimaux des divers modules (CP, P) de détection au sein desdites calottes (10) du châssis pour que l'axe (A) d'imagerie passe par la source dudit rayonnement gamma dans ledit objet (O) à imager.

2. Système selon la revendication 1, **caractérisé en ce qu'**il comporte au moins un moteur (M), piloté par ladite unité de traitement (5) et contrôlant la mobilité d'au moins un desdits modules (CP, P) de détection ledit châssis et/ou de l'objet à observer à l'intérieur dudit volume pour obtenir lesdites orientations et/ou lesdits emplacements optimaux définis par ladite unité de traitement (5).

3. Système selon une des revendications 1 et 2, **caractérisé en ce qu'**au moins un desdits ensembles de modules (P, CP) de détection comporte en fait seulement deux modules, grâce à un premier module (CP), dit "hybride", dont le scintillateur (2) comprend au moins une plaque de cristal scintillateur (P1), dit rapide, dont le temps de montée au pic de lumière est inférieur à 1ns, ledit module "hybride" étant apte à produire à la fois une diffusion Compton et une absorption d'au moins une partie du rayonnement gamma pour une détection de coïncidence entre les événements dans ce premier module hybride (CP) et les événements dans un second module (CP, P) de détection avec lequel ce premier module hybride (CP) forme donc ledit couple de modules PET de détection de coïncidences.

4. Système selon la revendication 3, **caractérisé en ce que** ledit second module (CP, P) de détection de chaque ensemble est soit un module hybride (CP) également, soit un module (P), dit "PET", apte à produire seulement une absorption photoélectrique pour une détection de coïncidence d'événements par rapport à l'autre module de détection avec lequel il forme ledit couple de modules PET de détection de coïncidences.

5. Système selon l'une des revendications 2 à 4, **caractérisé en ce que** chaque ensemble comporte plusieurs seconds modules (CP, P) en vis-à-vis du premier module hybride (CP) qui est orientable par ledit moteur (M) pour aligner l'axe (A) d'imagerie entre ledit module hybride (CP) et l'un ou l'autre de ces seconds modules (CP, P).

6. Système selon une des revendications 2 à 4, **caractérisé en ce que** ledit moteur (M) est apte à déplacer au moins ledit second module (CP, P) sur ledit châssis (1), pour le placer en vis-à-vis du premier module hybride (CP) qui est orienté par ledit moteur (M) pour aligner l'axe (A) d'imagerie entre le premier et le second module.

7. Système selon les revendications 2, 3 et 4, **caractérisé en ce que** le premier et le second module sont tous les deux des modules hybrides (CP) dont au moins un d'entre eux est orientable et au moins un d'entre eux est déplaçable sur le châssis (1), par ledit moteur (M).

8. Système selon l'une des revendications 1 à 7, **caractérisé en ce que** ladite unité (5) de traitement du signal analyse les signaux issus de chaque ensemble de modules (CP, P) de détection et détermine à la fois les événements de diffusion Compton et les événements d'absorption photoélectrique, pour obtenir la position tridimensionnelle, l'énergie et la séquence temporelle des interactions, Compton et photoélectriques, du rayonnement gamma dans le volume entre les modules (CP, P) de détection de chaque ensemble.

9. Système selon une des revendications 1 à 8, **caractérisé en ce que** l'unité de traitement calcule le temps de vol des photons entre les deux modules du couple de module PET premier module hybride (CP) et le second module (CP, P) pour mesurer la profondeur de la source de rayonnement gamma dans l'objet (O) à imager.

10. Système selon l'une des revendications 1 à 9, **caractérisé en ce que** l'unité de traitement utilise le signal d'au moins un couple module de type caméra Compton pour combiner leurs imageries Compton et réaliser une triangulation afin de mesurer la profondeur de la source de rayonnement gamma dans l'objet (O) à imager.

11. Système selon l'une des revendications 1 à 10, **caractérisé en ce que** :
- ledit scintillateur (2) est un cristal scintillateur monolithique ;
- ledit photo-détecteur (3) est un photo-détecteur (3) analogique dont le signal analogique fournit une mesure représentative de l'énergie des photons émis par ledit scintillateur (2) monolithique au cours du temps ;
- ledit dispositif d'acquisition (4) est configuré pour la détection et le filtrage des événements de scintillation grâce à un algorithme lui permettant de mesurer un premier temps (T1) auquel ledit signal analogique dépasse un premier seuil (S1) représentatif du premier photon détecté et de mesurer un second temps (T2) auquel ledit signal analogique dépasse un second seuil (S2) représentatif d'un nombre n de photons détectés, puis de calculer le délai (ΔT2) qui sépare le premier (T1) et le second (T2) temps.

12. Système selon la revendication 11, **caractérisé en ce que** ledit dispositif d'acquisition (4) recueille le signal du scintillateur (2) sur une pluralité (i) de pixels et compare le temps (T1) du premier photon détecté sur chacun des i pixels, afin de déterminer la valeur minimum de ce temps sur l'ensemble des pixels et d'estimer ainsi le temps (T0) d'arrivée du premier photon émis par le scintillateur.

13. Système selon l'une des revendications 11 et 12, **caractérisé en ce que** ledit dispositif d'acquisition (4) calcule une pente de montée du signal entre les deux seuils et élimine les événements détectés dont la pente de montée est inférieure à une valeur déterminée, afin d'éliminer le bruit du signal et ne conserver que des événements de scintillation validés.

14. Système selon l'une des revendications 11 à 13, **caractérisé en ce que** ladite unité de traitement (5) établit une carte du temps d'arrivée des photons à partir des événements filtrés par ledit dispositif d'acquisition (4) pour fournir une première estimation de la position d'un événement de scintillation et d'un disque de photons non diffusés, puis compare au moins un modèle de loi d'émission des photons par le scintillateur (2) avec le nombre de photons détectés dans ce disque à différents temps après l'arrivée du premier photon, afin de corriger en conséquence le diamètre de ce disque et donc d'améliorer la précision de la mesure de la profondeur de l'événement.

15. Système selon l'une des revendications 11 à 14, **caractérisé en ce que** l'unité de traitement corrige le retard probable entre les valeurs mesurées et réelles du temps d'arrivée du premier photon (T0), en comparant ces valeurs mesurées avec au moins un modèle de lois d'émissions des photons par le scintillateur (2), de préférence en tenant compte des mesures des temps d'arrivée du premier et un nième photon dans les pixels adjacents.

## Patentansprüche

1. System zur Abbildung durch Erkennung von Gammastrahlung, umfassend mindestens eine Verarbeitungseinheit (5), mindestens ein Gestell (1) und mindestens eine Menge von Erkennungsmodulen (CP, P), die auf dem mindestens einen Gestell (1) montiert sind und mindestens ein Signal bereitstellen, das von der mindestens einen Verarbeitungseinheit (5) analysiert wird, wobei jedes der Module mindestens einen Szintillator (2), der Photonen emittiert, wenn er der Einwirkung der Gammastrahlung ausgesetzt wird, mindestens einen Photodetektor (3), der das Signal (S) als Reaktion auf die Photonen, die von dem Szintillator (2) emittiert werden, erzeugt, und mindestens eine Erfassungsvorrichtung (4), die das Signal aufnimmt, um es an die Verarbeitungseinheit (5) zu übertragen, umfasst, wobei das System **dadurch gekennzeichnet ist, dass**:
- das Gestell (1) eine Vielzahl von Stellen umfasst, die entlang mindestens einem Abschnitt von zwei kugelförmigen Kappen (10) einander gegenüberliegend verteilt sind und ein Abbildungsvolumen im Inneren des Systems definieren, wobei die Kappen (10) durch einen Abstand (D) getrennt sind, der ermöglicht, einen abzubildenden Gegenstand (O) in dem Volumen aufzunehmen;
- das Gestell (1) auf mindestens einer Seite zwischen den zwei Kappen (10) offen ist, um freien Zugang zu dem abzubildenden Gegenstand (O) in dem Volumen zu gewähren;
- die Menge von Erkennungsmodulen (CP, P) mindestens ein Modul vom Compton-Kamera-Typ umfasst, das über ein Blickfeld (CV) verfügt, das zwischen die zwei Kappen (10) gerichtet ist, und deren Szintillator (2) mindestens eine so genannte schnelle Szintillator-Kristallplatte (P1) umfasst, deren Zeit zum Anstieg zum Lichtspitzenwert weniger als 1 ns beträgt, so dass dieses Modul dazu fähig ist, ein Bild unter Verwendung des Compton-Effekts zu produzieren;
- die Menge von Erkennungsmodulen (CP, P) mindestens ein Paar von PET-Koinzidenzerkennungsmodulen umfasst, die einander diametral auf dem Gestell jeweils auf einer der zwei Kappen (10) gegenüberliegen und eine Abbildungsachse (A) definieren;
- die Verarbeitungseinheit (5) das Signal, das von dem Modul vom Compton-Typ hervorgeht, analysiert, um den Schnittpunkt der Abbildungsachse (A) mit dem Blickfeld (CV) zu bestimmen und die Ausrichtungen und/oder optimalen Stellen von verschiedenen Erkennungsmodulen (CP, P) innerhalb der Kappen (10) des Gestells zu bestimmen, damit die Abbildungsachse (A) durch die Quelle der Gammastrahlung in dem abzubildenden Gegenstand (O) verläuft.

2. System nach Anspruch 1, **dadurch gekennzeichnet, dass** es mindestens einen Motor (M) umfasst, der von der Verarbeitungseinheit (5) gelenkt wird und die Beweglichkeit von mindestens einem der Erkennungsmodule (CP, P) des Gestells und/oder des zu beobachtenden Gegenstands im Inneren des Volumens steuert, um die Ausrichtungen und/oder die optimalen Stellen, die von der Verarbeitungseinheit (5) definiert wurden, zu erhalten.

3. System nach einem der Ansprüche 1 und 2, **dadurch gekennzeichnet, dass** mindestens eine der Mengen von Erkennungsmodulen (P, CP) tatsächlich dank eines ersten, so genannten "Hybrid"-Moduls (CP) nur zwei Module umfasst, dessen Szintillator (2) mindestens eine so genannte schnelle Szintillator-Kristallplatte (P1) umfasst, deren Zeit zum Anstieg zum Lichtspitzenwert weniger als 1 ns beträgt, wobei das "Hybrid"-Modul dazu fähig ist, gleichzeitig einen Compton-Effekt und eine Absorption von mindestens einem Teil der Gammastrahlung für eine Koinzidenzerkennung zwischen den Ereignissen in diesem ersten Hybrid-Modul (CP) und den Ereignissen in einem zweiten Erkennungsmodul (CP, P), mit dem dieses erste Hybrid-Modul (CP) somit das Paar von PET-Koinzidenzerkennungsmodulen bildet, zu produzieren.

4. System nach Anspruch 3, **dadurch gekennzeichnet, dass** das zweite Erkennungsmodul (CP, P) jeder Menge entweder ebenfalls ein Hybrid-Modul (CP) oder ein so genanntes "PET"-Modul (P) ist, das dazu fähig ist, nur eine photoelektrische Absorption für eine Koinzidenzerkennung von Ereignissen in Bezug auf das andere Erkennungsmodul, mit dem es das Paar von PET-Koinzidenzerkennungsmodulen bildet, zu produzieren.

5. System nach einem der Ansprüche 2 bis 4, **dadurch gekennzeichnet, dass** jede Menge mehrere zweite Module (CP, P) gegenüber dem ersten Hybrid-Modul (CP) umfasst, das durch den Motor (M) ausrichtbar ist, um die Abbildungsachse (A) zwischen dem Hybrid-Modul (CP) und dem einen oder dem anderen dieser zweiten Module (CP, P) auszurichten.

6. System nach einem der Ansprüche 2 bis 4, **dadurch gekennzeichnet, dass** der Motor (M) dazu fähig ist, mindestens das zweite Modul (CP, P) auf dem Gestell (1) zu verlagern, um es gegenüber dem ersten Hybrid-Modul (CP) zu platzieren, das von dem Motor (M) ausgerichtet wird, um die Abbildungsachse (A) zwischen dem ersten und dem zweiten Modul auszurichten.

7. System nach den Ansprüchen 2, 3 und 4, **dadurch gekennzeichnet, dass** das erste und das zweite Modul beide Hybrid-Module (CP) sind, von denen mindestens eines dieser ausrichtbar ist und mindestens eines dieser auf dem Gestell (1) durch den Motor (M) verlagerbar ist.

8. System nach einem der Ansprüche 1 bis 7, **dadurch gekennzeichnet, dass** die Verarbeitungseinheit (5) für das Signal die Signale, die von jeder Menge von Erkennungsmodulen (CP, P) hervorgehen, analysiert und gleichzeitig die Compton-Effekt-Ereignisse und die photoelektrischen Absorptionsereignisse bestimmt, um die dreidimensionale Position, die Energie und die zeitliche Abfolge von Compton- und photoelektrischen Wechselwirkungen der Gammastrahlung in dem Volumen zwischen den Erkennungsmodulen (CP, P) jeder Menge zu erhalten.

9. System nach einem der Ansprüche 1 bis 8, **dadurch gekennzeichnet, dass** die Verarbeitungseinheit die Flugzeit von Photonen zwischen den zwei Modulen des PET-Modulpaars von dem ersten Hybrid-Modul (CP) und dem zweiten Modul (CP, P) berechnet, um die Tiefe der Gammastrahlungsquelle in dem abzubildenden Gegenstand (O) zu messen.

10. System nach einem der Ansprüche 1 bis 9, **dadurch gekennzeichnet, dass** die Verarbeitungseinheit das Signal von mindestens einem Modulpaar vom Compton-Kamera-Typ verwendet, um ihre Compton-Abbildungen zu kombinieren und eine Triangulation umzusetzen, um die Tiefe der Gammastrahlungsquelle in dem abzubildenden Gegenstand (O) zu messen.

11. System nach einem der Ansprüche 1 bis 10, **dadurch gekennzeichnet, dass**:
- der Szintillator (2) ein monolithischer Kristall-Szintillator ist;
- der Photodetektor (3) ein analoger Photodetektor (3) ist, dessen analoges Signal eine Messung bereitstellt, die für die Energie von Photonen repräsentativ ist, die von dem monolithischen Szintillator (2) im Laufe der Zeit emittiert werden;
- die Erfassungsvorrichtung (4) für die Erkennung und die Filterung von Szintillationsereignissen dank eines Algorithmus konfiguriert ist, der ermöglicht, eine erste Zeit (T1) zu messen, zu der das analoge Signal einen ersten Grenzwert (S1) überschreitet, der für das erste erkannte Photon repräsentativ ist, und eine zweite Zeit (T2) zu messen, zu der das analoge Signal einen zweiten Grenzwert (S2) überschreitet, der für eine Anzahl n von erkannten Photonen repräsentativ ist, um die Zeitspanne (ΔT2) zu berechnen, die die erste (T1) und die zweite (T2) Zeit trennt.

12. System nach Anspruch 11, **dadurch gekennzeichnet, dass** die Erfassungsvorrichtung (4) das Signal des Szintillators (2) auf einer Vielzahl (i) von Pixeln aufnimmt und die Zeit (T1) des ersten Photons, das auf jedem von i Pixeln erkannt wird, vergleicht, um den minimalen Wert dieser Zeit auf der Menge von Pixeln zu bestimmen und somit die Zeit (T0) der Ankunft des ersten Photons, das von dem Szintillator emittiert wird, zu schätzen.

13. System nach einem der Ansprüche 11 und 12, **dadurch gekennzeichnet, dass** die Erfassungsvorrichtung (4) einen Anstiegsgradienten des Signals zwischen den zwei Grenzwerten berechnet und die erkannten Ereignisse eliminiert, deren Anstiegsgradient kleiner als ein bestimmter Wert ist, um das Rauschen des Signals zu eliminieren und nur validierte Szintillationsereignisse zu bewahren.

14. System nach einem der Ansprüche 11 bis 13, **dadurch gekennzeichnet, dass** die Verarbeitungseinheit (5) eine Karte der Ankunftszeit von Photonen aus Ereignissen, die von der Erfassungsvorrichtung (4) gefiltert wurden, erstellt, um einen ersten Schätzwert der Position eines Szintillationsereignisses und einer Scheibe von nicht gestreuten Photonen bereitzustellen, dann mindestens ein Modell eines Gesetzes der Emission von Photonen durch den Szintillator (2) mit der Anzahl von Photonen, die in dieser Scheibe zu verschiedenen Zeiten nach der Ankunft des ersten Photons erkannt wurden, vergleicht, um dementsprechend den Durchmesser dieser Scheibe zu korrigieren und somit die Präzision der Messung der Tiefe des Ereignisses zu verbessern.

15. System nach einem der Ansprüche 11 bis 14, **dadurch gekennzeichnet, dass** die Verarbeitungseinheit die wahrscheinliche Verzögerung zwischen den gemessenen und den tatsächlichen Werten der Ankunftszeit des ersten Photons (T0) korrigiert, indem sie diese gemessenen Werte mit mindestens einem Modell von Gesetzen der Emissionen von Photonen durch den Szintillator (2) vergleicht, vorzugsweise indem sie Messungen von Ankunftszeiten des ersten und eines n-ten Photons in den angrenzenden Pixeln zählt.

## Claims

1. System of imaging by gamma radiation detection comprising at least one processing unit (5), at least one chassis (1), and at least one set of detection modules (CP, P) mounted on said at least one chassis (1) and providing at least one signal analysed by said at least one processing unit (5), each of the modules comprising at least one scintillator (2) that emits photons, when it is subject to the influence of said gamma radiation, at least one photodetector (3) that generates said signal (S) in response to said photons emitted by said scintillator (2) and at least one acquisition device (4) which collects said signal in order to transmit it to said processing unit (5), the system being **characterised in that**:
- said chassis (1) comprises a plurality of locations distributed over at least one portion of two spherical caps (10) opposite each other defining an imaging volume within the system, said caps (10) being separated by a distance (D) that makes it possible to accommodate an object (O) to be imaged in said volume;
- said chassis (1) being open, on at least one side between the two caps (10), so as to allow free access to said object (O) to be imaged in said volume;
- said set of detection modules (CP, P) comprises at least one module of the Compton camera type, having a field of view (CV) directed between the two caps (10), of which the scintillator (2) comprises at least one so-called fast scintillator crystal plate (P1), of which the rise time to peak light is less than 1 ns, so that this module is capable of producing an image using Compton scattering;
- said set of detection modules (CP, P) comprises at least one pair of PET modules for detecting coincidences, diametrically opposed to each other on said chassis, each on one of the two caps (10), defining an imaging axis (A);
- said processing unit (5) analyses the signal from said Compton-type module to determine the intersection of said imaging axis (A) with said field of view (CV) and determine the orientations and/or optimum locations of the various detection modules (CP, P) within said caps (10) of the chassis so that the imaging axis (A) passes through the source of said gamma radiation in said object (O) to be imaged.

2. System according to claim 1, **characterised in that** it comprises at least one motor (M), driven by said processing unit (5) and controlling the mobility of at least one of said detection modules (CP, P) on said chassis and/or of the object to be observed within said volume in order to obtain said orientations and/or said optimum locations defined by said processing unit (5).

3. System according to one of claims 1 and 2, **characterised in that** at least one of said sets of detection modules (P, CP) in fact comprises only two modules, owing to a first so-called "hybrid" module (CP) of which the scintillator (2) comprises at least one so-called fast scintillator crystal plate (P1), of which the rise time to peak light is less than 1 ns, said "hybrid" module being capable of producing at the same time Compton scattering and absorption of at least a portion of the gamma radiation for detection of coincidence between the events in this first hybrid module (CP) and the events in a second detection module (CP, P) with which this first hybrid module (CP) therefore forms said pair of PET modules for detection of coincidences.

4. System according to claim 3, **characterised in that** said second detection module (CP, P) of each set is either a hybrid module (CP) as well, or a so-called "PET" module (P), capable of producing only photoelectric absorption for detection of coincidence of events relative to the other detection module with which it forms said pair of PET modules for detection of coincidences.

5. System according to one of claims 2 to 4, **characterised in that** each set comprises several second modules (CP, P) opposite the first hybrid module (CP) which is orientable by said motor (M) in order to align the imaging axis (A) between said hybrid module (CP) and one or the other of these second modules (CP, P).

6. System according to one of claims 2 to 4, **characterised in that** said motor (M) is capable of displacing at least said second module (CP, P) on said chassis (1), in order to place it opposite the first hybrid module (CP) which is oriented by said motor (M) in order to align the imaging axis (A) between the first and second modules.

7. System according to claims 2, 3 and 4, **characterised in that** the first and second modules are both hybrid modules (CP) of which at least one of them is orientable and at least one of them is displaceable on the chassis (1), by said motor (M).

8. System according to one of claims 1 to 7, **characterised in that** said signal processing unit (5) analyses the signals from each set of detection modules (CP, P) and simultaneously determines the Compton scattering events and the photoelectric absorption events, in order to obtain the three-dimensional position, the energy and the time sequence of interactions, Compton and photoelectric, of gamma radiation in the volume between the detection modules (CP, P) of each set.

9. System according to one of claims 1 to 8, **characterised in that** the processing unit calculates the time of flight of photons between the two modules of the pair of PET modules, the first hybrid module (CP) and the second module (CP, P), in order to measure the depth of the source of gamma radiation in the object (O) to be imaged.

10. System according to one of claims 1 to 9, **characterised in that** the processing unit uses the signal of at least one module pair of the Compton camera type to combine their Compton imaging and carry out triangulation in order to measure the depth of the source of gamma radiation in the object (O) to be imaged.

11. System according to one of claims 1 to 10, **characterised in that**:
- said scintillator (2) is a monolithic scintillator crystal;
- said photodetector (3) is an analogue photodetector (3) of which the analogue signal provides a measurement representing the energy of photons emitted by said monolithic scintillator (2) over time;
- said acquisition device (4) is configured for the detection and filtering of scintillation events owing to an algorithm allowing it to measure a first time (T1) at which said analogue signal exceeds a first threshold (S1) representing the first photon detected and to measure a second time (T2) at which said analogue signal exceeds a second threshold (S2) representing a number n of photons detected, then to calculate the time lag (ΔT2) which separates the first (T1) and second (T2) times.

12. System according to claim 11, **characterised in that** said acquisition device (4) collects the signal of the scintillator (2) over a plurality (i) of pixels and compares the time (T1) of the first photon detected on each of the i pixels, in order to determine the minimum value of this time over all of the pixels and so estimate the arrival time (T0) of the first photon emitted by the scintillator.

13. System according to one of claims 11 and 12, **characterised in that** said acquisition device (4) calculates a gradient of rise of the signal between the two thresholds and eliminates the events detected of which the gradient of rise is lower than a given value, in order to eliminate signal noise and keep only validated scintillation events.

14. System according to one of claims 11 to 13, **characterised in that** said processing unit (5) establishes a chart of the arrival time of photons on the basis of the events filtered by said acquisition device (4) in order to provide a first estimate of the position of a scintillation event and of a disc of unscattered photons, then compares at least one model law of emission of photons by the scintillator (2) with the number of photons detected in this disc at different times after arrival of the first photon, in order to therefore correct the diameter of this disc and so improve the precision of measurement of the depth of the event.

15. System according to one of claims 11 to 14, **characterised in that** the processing unit corrects the probable delay between the measured and actual values of the arrival time of the first photon (T0), by comparing these measured values with at least one model law of emission of photons by the scintillator (2), preferably taking into account the measurements of arrival times of the first and an nth photon in the adjacent pixels.
